**Europäisches Patentamt**

**European Patent Office**

**Office européen des brevets**

(19)

(11) Veröffentlichungsnummer : **0 128 565**
**B1**

(12) **EUROPÄISCHE PATENTSCHRIFT**

(45) Veröffentlichungstag der Patentschrift :
28.09.88

(51) Int. Cl.⁴ : **C 12 N 15/00, C 12 N 13/00**

(21) Anmeldenummer : 84106604.6

(22) Anmeldetag : 08.06.84

(54) Verfahren und Vorrichtung zur Fusion von Zellen.

(30) Priorität : 11.06.83 DE 3321238

(43) Veröffentlichungstag der Anmeldung :
19.12.84 Patentblatt 84/51

(45) Bekanntmachung des Hinweises auf die Patenterteilung : 28.09.88 Patentblatt 88/39

(84) Benannte Vertragsstaaten :
AT BE CH FR GB IT LI LU NL SE

(56) Entgegenhaltungen :
EP-A- 0 133 978
US-A- 4 001 197
BIOCHIMICA ET BIOPHYSICA ACTA, Band 772, 1984, Seiten 407-410, Elsevier Science Publishers B.V., NL; I. KRAMER et al.: "Magneto-electro-fusion of human erythrocytes"
JOURNAL OF MEMBRANE BIOLOGY, Band 67, 1982, Seiten 165-182, Springer-Verlag New York Inc., New York, US; U. ZIMMERMANN et al.: "Electric field-induced cell-to-cell fusion"
BIOCHIMICA ET BIOPHYSICA ACTA, Band 694, 1982, Seiten 227-277, Elsevier Biomedical Press, NL; U. ZIMMERMANN: "Electric field-mediated fusion and related electrical phenomena"

(73) Patentinhaber : **Kernforschungsanlage Jülich Gesellschaft mit beschränkter Haftung**
**Postfach 1913**
**D-5170 Jülich 1 (DE)**

(72) Erfinder : **Zimmermann, Ulrich, Prof.**
**Im Geyberg 21**
**D-5165 Hürtgenwald-Gey (DE)**

## Beschreibung

Die Erfindung bezieht sich auf ein Verfahren zur Fusion von Zellen, bei dem die in einer Zellsuspension im Fusionsraum befindlichen Zellen durch äußere Kräfte auf einen geringen Abstand zueinander gebracht werden und in der Membranstruktur der einander benachbarten Zellen durch äußere Einwirkungen Störungen verursacht werden, die zur Bildung von Membranbrücken zwischen benachbarten Zellen und zur Verschmelzung der Zellen führen.

Zwei Zellen in einer Suspension sollten, wenn sie sich berühren und ein enger Kontakt zwischen den Membranen beider Zellen entsteht, miteinander verschmelzen, da die Bausteine in der Membran beweglich sind. Eine derartige spontane Verschmelzung (Fusion) von Zellen wird unter natürlichen Bedingungen nicht oder nur äußerst selten beobachtet. Eine bekannte Ausnahme stellt die Befruchtung einer Eizelle durch eine Samenzelle bei der sexuellen Fortpflanzung dar. Die spontane Fusion wird durch die negative Ladung der Phospholipide und anderer Membrankomponenten behindert. Sie führt zur Abstoßung der Zellen, wenn sie sich auf einen geringen Abstand genähert haben. Zellverschmelzung erfordert aber, daß die beiden Membranen sich bis auf einen Abstand von weniger als $10^{-7}$ cm nähern können.

Die mit technischen Mitteln durchgeführte Fusion von Zellen ist in einem weiten Anwendungsbereich einsetzbar. So ist es für die biologischmedizinische Forschung von großem Interesse, eine große Zahl von Zellen miteinander zu verschmelzen. Bei geeigneter Größe der durch Fusion mehrerer oder gegebenenfalls vieler Zellen — beispielsweise 1 000 bis 10 000 Blutkörperchen — entstandenen großen Zellen lassen sich dann Mikroelektroden, Mikrodruckmeßsonden und andere Sensoren ohne irreversible Zerstörung der Membran in die große Zelle einführen. Die Technik, über die Sensoren direkt eine Reihe von Zellen und Membranfunktionen zu erfassen, ist dabei für die klinische Diagnostik, z. B. bei der Früherkennung von Erkrankungen sowie generell für die Grundlagenforschung von Bedeutung.

Die Technik der Fusion von Zellen kann außerdem eingesetzt werden für die Bildung von Hybridzellen durch Verschmelzung von zwei Zellen unterschiedlicher Herkunft. Dabei können Zellhybride aus Pflanzenzellen, aus denen wieder ganze Pflanzen gezüchtet werden können oder Zellhybride aus tierischen Zellen, über die monoklonale Antikörper, z. B. gegen Tumore und Leukämie, gewonnen werden können, gebildet werden. Als Beispiel sei genannt die Verschmelzung einer Lymphozytenzelle mit einer Myelomzelle, die besonders aus medizinischer und pharmazeutischer Sicht von großem Interesse ist. Bestimmte Lymphozyten bilden gegen Fremdstoffe im Organismus Antikörper, z. B. gegen ein Fremdeiweiß, das in die Blutbahn injiziert worden ist. Isoliert man die Lymphozyten und fusioniert sie mit einer Tumorzelle, wie der Myelomzelle, so besteht die Chance, daß sich eine sogenannte Hybridomzelle bildet, die die Eigenschaft beider Elternzellen besitzt. Diese Zelle produziert Antikörper, und zwar spezifisch nur gegen den betreffenden Fremdstoff (sogenannte monoklonale Antikörper). Sie ist unsterblich und läßt sich im Gegensatz zu einer normalen ausdifferenzierten Zelle, wie dem Lymphozyten, permanent in Nährmedien vermehren.

Ein Verfahren zur Fusion von Zellen der eingangs bezeichneten Art ist aus Biochimica et Biophysica Acta, 694 (1982), 227 — 277 (Electric Field-Mediated Fusion And Related Electrical Phenomena, U. Zimmermann) bekannt. Bei diesem bekannten Verfahren — dessen Ablauf unter dem Mikroskop beobachtet werden kann — wird der Membrankontakt zwischen wenigstens zwei Zellen durch Anlegen eines alternierenden, schwach inhomogenen Feldes erzeugt. Durch das elektrische Feld werden, bedingt durch Polarisationsprozesse in der Zelle, Dipole erzeugt, die sich gegenseitig anziehen, wenn die Zellen während ihrer Wanderung im elektrischen Feld sich einander nähern (sogenannte Dielektrophorese). Nach der Bildung der Zellenreihe werden die Störungen in der Membranstruktur zwischen benachbarten Zellen durch einen elektrischen Durchbruchpuls ausgelöst (J. Membrane Biol. 67, 165 — 182 (1982), Electric Field-Induced Cell-to-Cell Fusion, U. Zimmermann and J. Vienken). Dabei werden — nach den bisherigen Modellvorstellungen — Löcher in der Membrankontaktzone benachbarter Zellen erzeugt, die zu einem zytoplasmatischen Kontinuum zwischen den beiden Zellen und zur Brückenbildung von Lipiden zwischen den Membranen der benachbarten Zellen führen. Die Lipidmoleküle ordnen sich nicht mehr in ihre ursprüngliche Membran ein. Sobald sich eine Brücke gebildet hat, kommt es aus energetischen Gründen zur Abrundung des entstandenen Gebildes, das aus den über die Lipidbrücken miteinander verbundenen Zellen besteht.

Bei der Durchführung der bekannten Verfahrensweise der Sammlung der Zellen durch Dielektrophorese ist es jedoch erforderlich, daß die Lösung, in der sich die Zellen während der Durchführung des Verfahrens befinden, möglichst wenig leitend ist, da sonst die Wärmeentwicklung zu hoch ist, was zu Turbulenzen und zur Zerstörung des engen Membrankontaktes zwischen einander benachbarten Zellen führen kann. Das ist insofern von Nachteil, als eine nur wenig leitfähige Lösung nicht oder wenig zellverträglich ist und die Zellen daher in einem wenig leitenden Medium Schädigungen erleiden können, was u. a. ihre Lebensdauer beeinträchtigt.

Es ist ferner bekannt, die elektrischen Abstoßungskräfte, die bei geringem Abstand zwischen den Membranen zweier Zellen auftreten, durch Einsatz bestimmter Chemikalien, wie Polyethylenglykol (PEG) oder inaktivierte Viren, auszuschalten. Sowohl Viren als auch PEG vernetzen die beiden Zellmembranen, so daß ein enger Membrankontakt entsteht. Gleichzeitig werden durch die Viren und das PEG Störungen in der Membranstruktur erzeugt, die durch Einstellen unphysiologischer Bedingungen, wie z. B. Zugabe hoher Konzentrationen von Calciumionen und Wahl eines sehr hohen oder niedrigen pH-Wertes, noch verstärkt werden. Diese Störungen bewirken, daß sich in der Membrankontaktzone Löcher bilden und es damit zur Ausbildung von Phospholipidbrücken zwischen den benachbarten Zellmembranen kommt. Dies führt zur Verschmelzung der beiden Zellen unter Ausbildung einer neuen Einheit.

Bei der Durchführung der zuletzt genannten bekannten Verfahren kann jedoch die Zahl der miteinander zu verschmelzenden Zellen nicht gesteuert werden. Einerseits führt eine zu geringe Zelldichte in der die Zellen enthaltenden Lösung praktisch zu keinen Fusionsprodukten, da die Zellen nicht in Kontakt zueinander kommen. Andererseits führt eine für eine Fusion hinreichende Zelldichte — die auch durch Zentrifugieren der die Zellen enthaltenden Lösung erreicht werden kann — in unkontrollierter Weise zu Produkten, die aus dem Zwei-, Drei-, Vier- oder Vielfachen von individuellen Zellen bestehen.

Insbesondere im Hinblick auf die Bildung von Hybridzellen durch Verschmelzung von nur zwei Zellen unterschiedlicher Herkunft sind die zuletzt genannten bekannten Verfahren nicht zufriedenstellend, denn Zellhybride, die eine neuartige Kombination von Eigenschaften aufweisen, müssen nach der Durchführung der bekannten Verfahren erst durch die Anwendung sehr zeitraubender Selektionsmethoden isoliert werden. Die Gewinnung von Zellhybriden aus Pflanzenzellen, aus denen wieder ganze Pflanzen gezüchtet werden können oder von Zellhybriden aus tierischen Zellen, über die monoklonale Antikörper, z. B. gegen Tumore und Leukämie, gewonnen werden können, erfordert eine Fusionstechnik, bei der jeweils nur zwei Zellen miteinander fusionieren.

Es ist Aufgabe der Erfindung, ein Verfahren zur Fusion von Zellen zu schaffen, bei dessen Durchführung sich die Zellen auch in einer höher leitenden Lösung befinden können, die Zellen aber dennoch von dem eigentlichen Verfahrensschritt der Fusion zunächst auf geringen Abstand zueinander gebracht werden.

Diese Aufgabe wird gemäß der Erfindung dadurch gelöst, daß die Zellen mit magnetischen Teilchen dotiert und einem den Fusionsraum durchdringenden inhomogenen magnetischen Feld derart ausgesetzt werden, daß sich die dotierten Zellen in geringem Abstand zueinander ansammeln, worauf zur Erzeugung der Störungen in der Membranstruktur der benachbarten Zellen die Zellen entweder dem Puls eines elektrischen Feldes von mindestens der Höhe der Durchbruchspannung oder Störungen in der Membranstruktur verursachenden Chemikalien, wie Polyethylenglycol, oder Störungen in der Membranstruktur verursachenden inaktivierten Viren, wie Sendai-Viren, ausgesetzt werden.

Der Verfahrensschritt des Sammelns der Zellen läuft, da er allein unter Einwirkung magnetischer Kräfte und ohne elektrischen Stromfluß erfolgt, ohne jegliche Wärmeentwicklung ab. Dabei können die Zellen auch in einer Lösung mit höherer Leitfähigkeit suspendiert sein, beispielsweise in dem für die Zellen vorgesehenen Nährmedium oder in einem Medium, dessen Kalium-Konzentration gleich der Ionenkonzentration in der Zelle ist. Für den Fall, daß die Fusion der Zellen durch einen elektrischen Durchbruchpuls erfolgen soll, genügt dann — infolge der höheren Leitfähigkeit der Zellsuspension — eine Pulslänge im Bereich von Nano-Sekunden.

Durch die Verfahrensweise gemäß der Erfindung ist eine kontrollierte Fusion zweier oder einer vorbestimmten Anzahl von Zellen auch dann möglich, wenn Polyethylenglycol oder Sendai-Viren zur Störung der Membranstruktur eingesetzt werden. Die miteinander zu verschmelzende Zahl der Zellen kann dabei auch durch die vorgegebene Zelldichte in der Zellsuspension gesteuert werden. Darüberhinaus ist durch das Verfahren gemäß der Erfindung auch die Möglichkeit geschaffen worden, diese « nichtelektrischen » Fusionsverfahren im Hinblick auf die Dosierung der die Störungen bewirkenden Mittel genauer zu untersuchen bzw. zu optimieren. Das insbesondere auch deshalb, weil die Sammlung der Zellen am Ort der höchsten Felddichte bei Durchführung des Verfahrens gemäß der Erfindung unter dem Mikroskop beobachtet werden kann.

Zweckmäßig ist, daß die magnetischen Teilchen auf die Oberfläche der Zellen aufgebracht werden. Um die magnetischen Teilchen in möglichst feiner Verteilung auf die Oberfläche der Zellen aufzubringen und um außerdem zu vermeiden, daß sich in der Zellsuspension einzelne, nicht an der Oberfläche der Zellen adsorbierte magnetische Teilchen befinden, werden die Zellen zweckmäßigerweise zur Aufnahme der magnetischen Teilchen auf ihre Oberfläche in eine Lösung gegeben, die die magnetischen Teilchen kolloidal gelöst enthält. Nach einer gewissen Zeit, nach der der Adsorptionsvorgang weitgehend beendet ist, können die in der kolloidalen Lösung vorhandenen überschüssigen magnetischen Teilchen durch Abzentrifugieren der Zellen von diesen getrennt werden.

Es kann ferner zweckmäßig sein, daß die magnetischen Teilchen nicht an der Oberfläche der Zellen adsorbiert, sondern in diese eingebracht werden sollen. Dazu wird die Durchlässigkeit der Membran der Zellen, die sich in einer die magnetischen Teilchen in kolloidaler Form enthaltenden Lösung befinden, durch Anwendung eines elektrischen Feldpulses erhöht, so daß die magnetischen Teilchen in das Zellinnere gelangen. So werden beispielsweise durch eine Zellsuspension, die Erythrozyten und $Fe_3O_4$-Partikeln enthält, ein Feldpuls von etwa 15 kV/cm und 50 µs Dauer appliziert und damit eine hinreichende Permeabilitätserhöhung der Zellmembranen erzielt, um die $Fe_3O_4$-Teilchen in die Erythrozyten gelangen zu lassen. Nach Ausheilen der Permeabilitätserhöhung können die zusätzlich an der Oberfläche adsorbierten Magnetteilchen durch mehrmaliges Waschen der Zellen mit einer isotonen Lösung entfernt werden.

Zweckmäßigerweise werden als magnetische Teilchen ferromagnetische Teilchen, insbesondere $Fe_3O_4$-Partikeln, eingesetzt.

Für den Fall, daß eine besondere Ausrichtung der Zellen, insbesondere eine Aufreihung der Zellen in Form einer Perlenkette, gewünscht ist, werden die Zellen nach der Sammlung im Bereich der höchsten Felddichte des magnetischen Feldes einem äußeren, inhomogenen elektrischen Feld einer Frequenz im Bereich von 5 kHz bis 2 MHz und einer Stärke im Bereich von 10 V/cm bis 2 000 V/cm je nach Größe der Zellen für kurze Zeit — im allgemeinen einige Sekunden — derart ausgesetzt, daß sich die Zellen in der vorbestimmten Ausrichtung aneinanderreihen. Da die Zellen vor Anwendung des elektrischen Wechselfeldes bereits durch die Einwirkung des magnetischen Feldes gesammelt worden waren, bedarf es nur einer kurzen Einwirkung des elektrischen Feldes, um die gewünschte Aufreihung der Zellen zu erreichen. Eine Erwärmung der Zellsuspension und damit der Zellen ist damit — bei gleichzeitigem Erzielen einer Zellenreihe — weitgehend vermieden.

Für den Fall, daß die Störungen in der Membranstruktur durch Verwendung von Polyethylenglycol oder Sendai-Viren bewirkt werden, wird zweckmäßigerweise zunächst das Mittel der die Zellen enthaltenden Suspension zugegeben, danach die Zellen gesammelt oder gesammelt und anschließend in Reihen ausgerichtet und sodann Calciumionen zur Bewirkung der Störungen in der Membran der Zellen zugegeben. Bei Verwendung von Sendai-Viren wird außerdem die Temperatur auf 37° C erhöht. Bei dieser Verfahrensweise setzt die Wirkung der Mittel erst nach Ausrichtung der Zellen ein.

Für die Durchführung des Verfahrens gemäß der Erfindung unter Verwendung von Störungen in der Membranstruktur bewirkenden Chemikalien oder Viren ist eine Vorrichtung geeignet, bei der eine Kammer mit einem aus elektrisch nichtleitenden Wänden gebildeten Raum zur Aufnahme einer Zellsuspension vorgesehen ist und bei der ein Magnet derart an der Kammer angeordnet ist, daß der Raum der Kammer von einem inhomogenen magnetischen Feld durchdrungen wird. Als Magnet kann ein Permanentmagnet, jedoch auch ein Elektromagnet oder eine Kombination von beiden Verwendung finden, mit der man beispielsweise die magnetischen Feldkräfte ändern kann.

Für die Durchführung der Ausführungsvariante des Verfahrens gemäß der Erfindung, bei der Störungen in der Membranstruktur der Zellen durch einen elektrischen Durchbruchpuls bewirkt werden, ist eine Vorrichtung geeignet, bei der eine Kammer mit einem aus elektrisch nichtleitenden Wänden gebildeten Raum zur Aufnahme einer Zellsuspension vorgesehen ist, in den wenigstens zwei Elektroden derart hineinragen, daß ein zwischen den Elektroden begrenzter Bereich gebildet wird, in welchem die Zellen einem zwischen den Elektroden ausgebildeten elektrischen Feld ausgesetzt sind und bei dem ein Magnet an der Kammer derart angeordnet ist, daß der Raum der Kammer von einem inhomogenen magnetischen Feld durchdrungen wird.

Eine vorteilhafte Ausführungsform der Vorrichtung besteht bei beiden Vorrichtungsvarianten darin, daß der Magnet derart an der Kammer angeordnet ist, daß das den Raum durchdringende magnetische Feld mit seiner höchsten Dichte einem in den Raum der Kammer hineinragenden Teil des Magneten entspringt. Das in den Raum hineinragende Teil des Magneten ist dabei zweckmäßigerweise eine Spitze oder Kante aufweisender Polschuh. Der Polschuh kann dabei zugleich eine der Elektroden sein.

Ausführungsvarianten der Vorrichtung gemäß der Erfindung sind in der Zeichnung schematisch dargestellt und werden im folgenden näher erläutert :

Es zeigen

Figur 1 den Kammerraum zur Aufnahme der Zellsuspension mit beidseitig des Raumes angeordneten Magnetpolen,

Figur 2 den Kammerraum mit nur auf einer Seite des Raumes angeordnetem Magneten,

Figur 3 Anordnung des Magneten gemäß Figur 1 mit in den Kammerraum hineinragenden Elektroden,

Figur 4 Anordnung des Magneten gemäß Figur 1 mit in den Kammerraum hineinragendem und zugleich als Elektrode ausgebildetem Teil des Magneten,

Figur 5 Anordnung des Magneten gemäß Figur 1, bei der beide Magnetpole in den Kammerraum hineinragen und als Elektroden ausgebildet sind,

Figur 6a Draufsicht auf eine Kammer zur Behandlung der Zellen im elektrischen Feld mit in den Kammerraum hineinragendem Polschuh des Magneten,

Figur 6b Längsschnitt durch die Kammer gemäß Figur 6a entlang der Linie A-B.

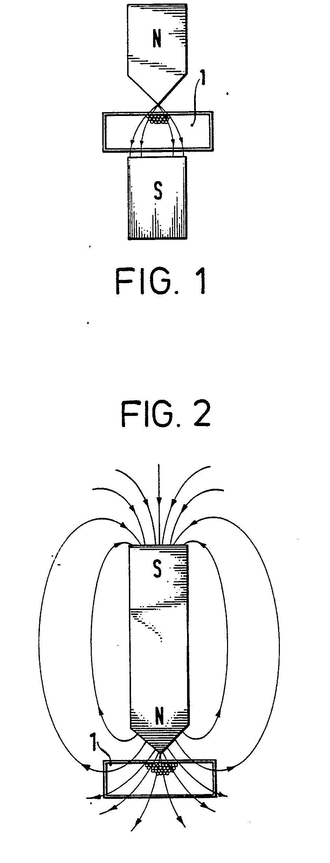

Die in Figur 1 und Figur 2 gezeigten Anordnungen dienen der Durchführung derjenigen Verfahrensvarianten, bei denen der Durchbruch der Zellen durch chemische Mittel oder durch inaktivierte Viren bewirkt wird. Der Kammerraum 1 ist dabei entweder oben offen oder geschlossen, wobei im letzteren Fall eine Möglichkeit zum Füllen des Kammerraumes mit Zellsuspension, beispielsweise eine Zu- und Ableitung, vorgesehen ist.

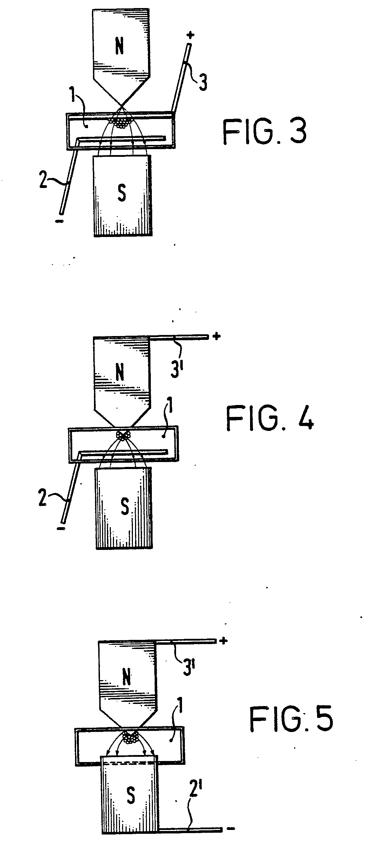

Bei der in Figur 3 dargestellten Anordnung ragen in den Kammerraum zwei Elektroden 2 und 3 hinein, deren Abstand je nach Art und Größe der zu behandelnden Zellen im allgemeinen zwischen 5 μm und 1 000 μm liegt. Die dem Nordpol des Magneten, dem das magnetische Feld mit hoher Felddichte entspringt, zugewandte Elektrode 3 liegt direkt an der dem Nordpol zugewandten Kammerwand an oder ist als diese ausgebildet. Zu dieser Elektrode 3' gelangen die Zellen, die sich auf den Ort höchster magnetischer Felddichte zu bewegen. Die Zellen sammeln sich dabei in dichter Packung und können durch einen über die Elektroden geleiteten elektrischen Puls in der Höhe der Durchbruchsspannung fusioniert werden. Die Elektroden sind dabei im allgemeinen an eine — in der Zeichnung nicht dargestellte — Einrichtung zur Erzeugung eines Rechteckpulses (für den elektrischen Durchbruch) bis 300 V angeschlossen.

Sollen die Zellen nach der Sammlung im magnetischen Feld und vor der Fusionierung in Form einer Perlenkette aneinandergereiht werden, so ist an die Elektroden im allgemeinen eine Einrichtung zur Erzeugung eines elektrischen Wechselfeldes mit einer Ausgangsspannung von 50 V angeschlossen.

Bei der in Figur 4 dargestellten Anordnung ragt ein Teil des Magneten — ein eine Spitze aufweisender Polschuh — in den Kammerraum 1 hinein und ist zugleich als Elektrode 3' ausgebildet. Bei dieser Ausführungsform werden die Zellen direkt an der Spitze der Elektrode 3' gesammelt.

Eine Ausführungsform der Vorrichtung, bei der sowohl Nord- als auch Südpol des Magneten in den Kammerraum 1 hineinragen und zugleich als Elektroden 2' und 3' ausgebildet sind, zeigt Figur 5.

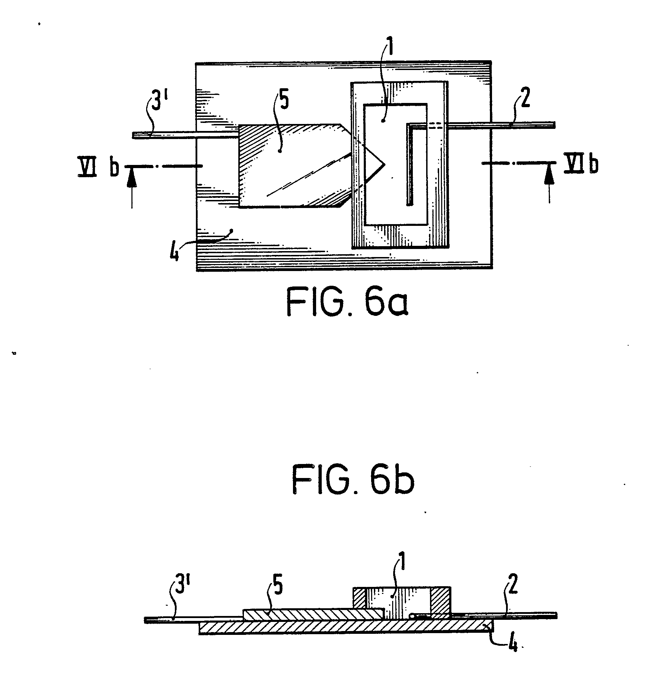

In Figur 6a und 6b ist eine besondere Ausführungsform einer Kammer zur elektrischen Behandlung von Zellen dargestellt. Der Raum 1 der Kammer wird begrenzt durch eine Grundplatte 4 und ein die seitlichen Wände des Raumes bildendes Seitenteil 5. Zur oberen Begrenzung des Raumes kann eine Deckplatte oder eine Folie vorgesehen sein.

In den Raum 1 ragen zwei Elektroden 2 und 3' hinein, von denen die Elektrode 3' plattenförmig und aus einem Material mit relativ großer magnetischer Permeabilität (Weicheisen) ausgebildet ist. Zur Durchführung des Verfahrens gemäß der Erfindung wird durch die Elektrode 3' — beispielsweise durch Kontakt mit einem Stabmagneten — der magnetische Kraftfluß geleitet, um so den gewünschten Verlauf der den Raum 1 durchdringenden magnetischen Kraftlinien zu erhalten.

Ausführungsbeispiel 1

An der Oberfläche von erythroleukämischen Zellen (Friend-Zellen) wurden $Fe_3O_4$-Partikeln (Magnetit) adsorbiert. Hierzu wurde 1 ml der Zellsuspension mit 100 μl einer kolloidalen isotonen $Fe_3O_4$-Lösung versetzt. Zur Erhöhung der Adsorption der $Fe_3O_4$-Partikeln wurde zusätzlich noch 1 mg Pronase (oder Dispase) zugegeben (die kolloidale $Fe_3O_4$-Lösung war durch Filtration einer $Fe_3O_4$-Aufschlämmung durch ein Membranfilter mit 0,45 μm Porendurchmesser hergestellt worden). Nach einer Inkubationszeit von etwa einer Stunde, nach der der Adsorptionsvorgang weitgehend abgeschlossen war, wurden die Zellen durch Abzentrifugieren aus der Lösung von den überschüssigen $Fe_3O_4$-Partikeln abgetrennt, da die $Fe_3O_4$-Partikeln in der kolloidalen Lösung nicht abzentrifugierbar sind. Die Zellen wurden anschließend in einer isotonen Lösung gewaschen.

Die mit den magnetischen Teilchen dotierten Zellen wurden in einer isotonen 0.3 molaren Mannitlösung inkubiert, der 41,5 % Polyethylenglycol (PEG ; MW 6 000) sowie 15 % Dimethylsulfoxid (DMSO) zugegeben wurden.

Die Zellen wurden in eine Fusionskammer der in Figur 1 dargestellten Art gegeben. Die Magnetfeldstärke des verwendeten Magneten betrug an dessen Oberfläche ca. 1 kG. (Wie sich gezeigt hat, konnte der Sammlungsvorgang jedoch bereits bei Verwendung eines Stückes magnetisierten Stahlbleches bei Erythrozyten, die mit $Fe_3O_4$-Partikeln dotiert worden waren, festgestellt werden.)

Nach Sammlung der Zellen am Ort der größten Felddichte wurde in den Kammerraum vorsichtig eine Lösung zugegeben, die 10 mM $CaCl_2$ in 0.3 molarer Mannitlösung enthielt. Durch die Zugabe der Calciumionen wurde die Fusion der aneinanderliegenden Zellen durch PEG ausgelöst.

Ausführungsbeispiel 2

Entsprechend Ausführungsbeispiel 1 wurden Avena-Sativa-Protoplasten mit Magnetitteilchen dotiert und mit PEG fusioniert.

Ausführungsbeispiel 3

Wie in Ausführungsbeispiel 1 beschrieben, wurden erythroleukämische Zellen mit Magnetitteilchen dotiert und im magnetischen Feld gesammelt. In der die Zellen enthaltenden Lösung war jedoch kein PEG, sondern inaktivierte Sendai-Viren einer Konzentration von etwa $2 \times 10^3$ HAU/ml (haemagglutinierende Einheiten) enthalten. Nach Sammlung der Zellen im magnetischen Feld wurde die doppelte Menge einer Lösung zugegeben, die etwa 4 mM/l $CaCl_2$ in phosphatgepufferter isotoner NaCl-Lösung enthielt. Darauf folgende Temperaturerhöhung auf 37 °C löste Fusion aus.

Ausführungsbeispiel 4

Wie in Ausführungsbeispiel 1 beschrieben, wurden Avena-Sativa-Protoplasten mit Magnetitteilchen dotiert und im magnetischen Feld gesammelt. Die Suspensionslösung enthielt 5 mM $CaCl_2$ und 1 mg Pronase/ml, was einer elektrischen Leitfähigkeit von $1 \times 10^{-3} \Omega^{-1} \times cm^1$ entspricht. Nach Sammlung der Zellen wurde ein Feldpuls von 2 400 V/cm und 500 ns Dauer appliziert und auf diese Weise die Zellen fusioniert.

Ausführungsbeispiel 5

Wie in Ausführungsbeispiel 1 beschrieben, wurden Avena-Sativa-Protoplasten mit Magnetitteilchen dotiert und im magnetischen Feld gesammelt. Die Suspensionslösung hatte die in Ausführungsbeispiel 4 angegebene Zusammensetzung.

Zur Ausbildung von Zellenreihen wurde ein elektrisches Wechselfeld einer Frequenz von 1 500 kHz und einer Stärke von 200 V/cm für 5 Sekunden angelegt. Hiernach wurde, wie in Ausführungsbeispiel 4 beschrieben, mittels eines elektrischen Feldpulses fusioniert.

Alle vorgenannten. Ausführungsbeispiele wurden mittels einer Kammer der in Figur 61/6b dargestellten Art durchgeführt, wobei allerdings bei den Ausführungsbeispielen 1 bis 3 eine elektrische Spannung nicht angelegt wurde.

**Patentansprüche**

1. Verfahren zur Fusion von Zellen, bei dem die in einer Zellsuspension im Fusionsraum befindlichen Zellen durch äußere Kräfte auf einen geringen Abstand zueinander gebracht werden und in der Membranstruktur benachbarter Zellen durch äußere Einwirkungen Störungen verursacht werden, die zur Bildung von Membranbrücken zwischen den benachbarten Zellen und zur Verschmelzung der Zellen führen, dadurch gekennzeichnet, daß die Zellen mit magnetischen Teilchen dotiert und einem den Fusionsraum durchdringenden. inhomogenen magnetischen Feld derart ausgesetzt werden, daß sich die dotierten Zellen in geringem Abstand zueinander ansammeln, worauf zur Erzeugung der Störungen in der Membranstruktur der benachbarten Zellen die Zellen entweder dem Puls eines elektrischen Feldes von mindestens der Höhe der Durchbruchspannung oder Störungen in der Membranstruktur verursachenden Chemikalien, wie Polyethylenglycol, oder Störungen in der Membranstruktur verursachenden inaktivierten Viren, wie Sendai-Viren, ausgesetzt werden.

2. Verfahren nach Anspruch 1, dadurch gekennzeichnet, daß die magnetischen Teilchen auf die Oberfläche der Zellen aufgebracht werden.

3. Verfahren nach Anspruch 1, dadurch gekennzeichnet, daß die magnetischen Teilchen in die Zellen eingebracht werden.

4. Verfahren nach Anspruch 2 oder 3, dadurch gekennzeichnet, daß die Zellen zur Aufnahme der magnetischen Teilchen in eine kolloidale, die magnetischen Teilchen enthaltende Lösung gegeben werden.

5. Verfahren nach einem der Ansprüche 1 bis 4, dadurch gekennzeichnet, daß die magnetischen Teilchen ferromagnetische Teilchen sind.

6. Verfahren nach Anspruch 5, dadurch gekennzeichnet, daß die magnetischen Teilchen $Fe_3O_4$-Partikeln sind.

7. Verfahren nach einem der Ansprüche 1 bis 6, dadurch gekennzeichnet, daß nach Sammlung der Zellen im Bereich der höchsten Felddichte des magnetischen Feldes die Zellen einem äußeren inhomogenen elektrischen Feld einer Frequenz im Bereich von 5 kHz bis 2 MHz und einer Stärke im Bereich von 10 V/cm bis 2 000 V/cm je nach Größe der Zellen für kurze Zeit derart ausgesetzt werden, daß sich die Zellen in vorbestimmter Ausrichtung aneinanderreihen.

8. Verfahren nach einem der Ansprüche 1 bis 7, dadurch gekennzeichnet, daß für den Fall, daß die Störungen in der Membranstruktur durch Verwendung von Polyethylenglycol bewirkt werden, zunächst Polyethylenglycol der die Zellen enthaltenden Suspension zugegeben wird, danach die Zellen im magnetischen Feld gesammelt oder gesammelt und aneinandergereiht werden und sodann Calciumionen zur Bewirkung der Störungen in der Membran der Zellen zugegeben werden.

9. Verfahren nach einem der Ansprüche 1 bis 7, dadurch gekennzeichnet, daß für den Fall, daß die Störungen in der Membranstruktur durch Verwendung von inaktivierten Sendai-Viren bewirkt werden, zunächst die Viren der die Zellen enthaltenden Suspension zugegeben werden, danach die Zellen im magnetischen Feld gesammelt oder gesammelt und aneinandergereiht werden und sodann Calciumionen zur Bewirkung der Störungen in der Membran der Zellen zugegeben werden.

10. Vorrichtung zur Durchführung des Verfahrens unter Verwendung von Störungen in der Membranstruktur bewirkenden Chemikalien oder Viren gemäß einem der Ansprüche 1 bis 9, bei der eine Kammer mit einem aus elektrisch nicht leitenden Wänden gebildeter Raum zur Aufnahme einer Zellsuspension vorgesehen ist, gekennzeichnet durch einen derart an der Kammer angeordneten Magneten, daß der Raum (1) der Kammer von einem inhomogenen magnetischen Feld durchdrungen wird.

11. Vorrichtung zur Durchführung des Verfahrens unter Verwendung von einem Störungen in der Membranstruktur bewirkendem elektrischen Durchbruchpuls gemäß einem der Ansprüche 1 bis 7, bei der eine Kammer mit einem aus elektrisch nicht leitenden Wänden gebildeter Raum zur Aufnahme einer Zellsuspension vorgesehen ist, in den wenigstens zwei Elektroden derart hineinragen, daß ein zwischen den Elektroden begrenzter Bereich gebildet wird, in welchem die Zellen einem zwischen den Elektroden ausgebildeten elektrischen Feld ausgesetzt sind, gekennzeichnet durch einen derart an der Kammer angeordneten Magneten, daß der Raum (1) der Kammer von einem inhomogenen magnetischen Feld durchdrungen wird.

12. Vorrichtung nach einem der Ansprüche 10 oder 11, dadurch gekennzeichnet, daß der Magnet derart an der Kammer angeordnet ist, daß das den Raum (1) durchdringende magnetische Feld mit seiner höchsten Dichte einem in den Raum der Kammer hineinragenden Teil des Magneten entspringt.

13. Vorrichtung nach Anspruch 12, dadurch gekennzeichnet, daß der in den Raum hineinragende Teil des Magneten ein eine Spitze oder Kante aufweisender Polschuh (3) ist.

14. Vorrichtung nach Anspruch 13, dadurch gekennzeichnet, daß der Polschuh (3) zugleich Elektrode ist.

15. Vorrichtung nach einem der Ansprüche 10 bis 14, dadurch gekennzeichnet, daß der Magnet ein Permanent- oder ein Elektromagnet ist.

16. Vorrichtung nach einem der Ansprüche 10 bis 14, dadurch gekennzeichnet, daß der Magnetaus einer Kombination aus einem Permanentmagneten und einem Elektromagneten besteht.

## Claims

1. Process for the fusion of cells, wherein the cells present in a cell suspension in the fusion space are brought to a small spacing from one another by external forces, and external influences cause in the membrane structure of adjacently situated cells disturbances which lead to the forming of membrane bridges between the adjacent cells and to the fusion of the cells, characterised in that the cells are doped with magnetic particles, and are so subjected to an unhomogeneous magnetic field penetrating the fusion space that the doped cells accumulate at small spacings from one another, whereupon, for producing disturbances in the membrane structure of adjacent cells, the cells are subjected either to the pulse of an electrical field of at least the magnitude of the disruptive voltage, or to chemicals causing disturbances in the membrane structure, such as polyethylene glycol, or to inactivated viruses causing disturbances in the membrane structure, such as Sendai viruses.

2. Process according to claim 1, characterised in that the magnetic particles are applied to the surface of the cells.

3. Process according to claim 1, characterised in that the magnetic particles are introduced into the cells.

4. Process according to claim 2 or 3, characterised in that, for taking-up the magnetic particles, the cells are put into a colloidal solution containing the magnetic particles.

5. Process according to one of claims 1 to 4, characterised in that the magnetic particles are ferromagnetic particles.

6. Process according to claim 5, characterised in that the magnetic particles are $Fe_3O_4$ particles.

7. Process according to one of claims 1 to 6, characterised in that after the collection of the cells in the region of the highest field density of the magnetic field the cells are for a short time so subjected to an external unhomogeneous electrical field having a frequency in the region of 5 kHz to 2 MHz and an intensity in the range of 10 V/cm to 2 000 V/cm, depending on the size of the cells, that the cells line up in a predetermined alignment.

8. Process according to one of claims 1 to 7, characterised in that in the event that the disturbances in the membrane structure are brought about by the use of polyethylene glycol, first polyethylene glycol is added to the suspension containing the cells, thereafter the cells are collected in the magnetic field or collected and lined up, and then calcium ions are added to effect the disturbances in the membranes of the cells.

9. Process according to one of claims 1 to 7, characterised in that in the event that the disturbances in the membrane structure are brought about by the use of inactivated Sendai viruses, first the viruses are added to the suspension containing the cells, thereafter the cells are collected in the magnetic field or collected and lined up, and then calcium ions are added to effect the disturbances in the membranes of the cells.

10. Device for carrying out the process, using chemicals or viruses for effecting disturbances in the membrane structure, according to one of claims 1 to 9, wherein a chamber is provided having a space which is formed by electrically non-conductive walls and which is adapted to receive a cell suspension, characterised by a magnet so arranged at the chamber that the space (1) of the chamber is permeated by an unhomogeneous magnetic field.

11. Device for carrying out the process, using an electrical disruptive pulse for effecting disturbances in the membrane structure, according to one of claims 1 to 7, wherein a chamber is provided having a space for accommodating a cell suspension, said space being formed by electrically non-conductive walls and at least two electrodes projecting into it in such a manner that a region defined between the electrodes is formed in which the cells are subjected to an electrical field produced between the electrodes, characterised by a magnet so arranged at the chamber that the space (1) of the chamber is permeated by an unhomogeneous magnetic field.

12. Device according to one of claims 10 or 11, characterised in that the magnet is so arranged at the chamber that the magnetic field permeating the space (1) at its highest density emanates from a magnet part which projects into the chamber space.

13. Device according to claim 12, characterised in that the part of the magnet which projects into the space is a pole piece (3) which has a pointed portion or edge.

14. Device according to claim 13, characterised in that the pole piece (3) is at the same time an electrode.

15. Device according to one of claims 10 to 14, characterised in that the magnet is a permanent magnet or an electromagnet.

16. Device according to one of claims 10 to 14, characterised in that the magnet consists of a combination of a permanent magnet and an electromagnet.

## Revendications

1. Procédé de fusion de cellules, dans lequel on met les cellules se trouvant dans une suspension de cellules dans la chambre de fusion, à une faible distance les unes des autres sous l'effet de forces extérieures, et on provoque, par des effets extérieurs, dans la texture de membranes de cellules voisines des perturbations qui entraînent la formation de ponts membraneux entre les cellules voisines et la fusion des cellules, caractérisé en ce qu'il consiste à doper les cellules de particules magnétiques et à les soumettre à un champ magnétique hétérogène passant dans la chambre de fusion, de sorte que les cellules dopées se rassemblent les unes avec les autres à une faible distance, les cellules étant soumises, pour produire les perturbations de la texture de membrane de cellules voisines, à l'impulsion d'un champ électrique ayant au moins la valeur de la tension de rupture, ou à des produits chimiques comme le polyéthylèneglycol provoquant des perturbations dans la texture de la membrane, ou à des virus inactivés comme des virus Sendai, provoquant des perturbations dans la texture de la membrane.

2. Procédé suivant la revendication 1, caractérisé en ce qu'il consiste à déposer les particules magnétiques à la surface des cellules.

3. Procédé suivant la revendication 1, caractérisé en ce qu'il consiste à incorporer les particules magnétiques aux cellules.

4. Procédé suivant la revendication 2 ou 3, caractérisé en ce qu'il consiste à charger les cellules, en vue de la réception des particules magnétiques, dans une solution colloïdale contenant les particules magnétiques.

5. Procédé suivant l'une des revendications 1 à 4, caractérisé en ce que les particules magnétiques sont des particules ferromagnétiques.

6. Procédé suivant la revendication 5, caractérisé en ce que les particules magnétiques sont des particules de $Fe_3O_4$.

7. Procédé suivant l'une des revendications 1 à 6, caractérisé en ce qu'il consiste, après l'accumulation des cellules dans la région de la plus grande densité du champ magnétique, à soumettre les cellules à un champ électrique extérieur hétérogène, d'une fréquence comprise entre 5 kHz et 2 MHz et d'une intensité de 10 V/cm à 2 000 V/cm, suivant la dimension des cellules, pendant des durées brèves de manière que les cellules s'alignent dans une direction déterminée à l'avance.

8. Procédé suivant l'une des revendications 1 à 7, caractérisé en ce qu'il consiste, dans le cas où les perturbations de la texture de la membrane sont provoquées par l'utilisation du polyéthylèneglycol, à ajouter d'abord du polyéthylèneglycol à la suspension contenant les cellules, puis à assembler les cellules dans le champ magnétique ou à les assembler et à les aligner, et à ajouter immédiatement des ions calcium pour provoquer les perturbations dans la membrane des cellules.

9. Procédé suivant l'une des revendications 1 à 7, caractérisé en ce qu'il consiste, dans le cas où les perturbations de la texture de la membrane sont provoquées en utilisant des virus Sendai inactivés, à charger d'abord les virus dans la suspension contenant les cellules, puis à assembler les cellules dans le champ magnétique ou à les assembler et à les aligner et à ajouter immédiatement des ions calcium pour provoquer les perturbations dans la membrane des cellules.

10. Installation pour la mise en oeuvre du procédé, en utilisant des produits chimiques ou des virus provoquant des perturbations dans la texture de la membrane suivant l'une des revendications 1 à 9, dans laquelle il est prévu une chambre dont l'intérieur est entouré de parois non conductrices de l'électricité et est destiné à la réception d'une suspension de cellules, caractérisée par un aimant disposé de telle façon, par rapport à la chambre, que passe à l'intérieur (1) de la chambre un champ magnétique hétérogène.

11. Installation pour la mise en oeuvre du procédé, en utilisant une impulsion électrique de rupture provoquant des perturbations dans la texture de la membrane suivant l'une des revendications 1 à 7, dans laquelle il est prévu une chambre dont l'intérieur est entouré de parois non conductrices de l'électricité, qui est destiné à la réception d'une suspension de cellules et dans lequel pénètre au moins deux électrodes de façon à former entre les électrodes une région délimitée dans laquelle les cellules sont soumises à un champ électrique formé entre les électrodes, caractérisée par un aimant disposé, par rapport à la chambre, de façon que passe à l'intérieur (1) de la chambre un champ magnétique hétérogène.

12. Installation suivant l'une des revendications 10 ou 11, caractérisée en ce que l'aimant est disposé, par rapport à la chambre, de façon que le champ magnétique passant à l'intérieur (1) provienne en ayant sa densité la plus grande, d'une partie de l'aimant, qui pénètre à l'intérieur de la chambre.

13. Installation suivant la revendication 12, caractérisée en ce que la partie de l'aimant, qui pénètre à l'intérieur est une pièce polaire (3) ayant un sommet ou une arête.

14. Installation suivant la revendication 13, caractérisée en ce que la pièce polaire (3) est en même temps une électrode.

15. Installation suivant l'une des revendications 10 à 14, caractérisée en ce que l'aimant est un aimant permanent ou un électroaimant.

16. Installation suivant l'une des revendications 10 à 14, caractérisée en ce que l'aimant est constitué d'une combinaison d'un aimant permanent et d'un électroaimant.

FIG. 1

FIG. 2

FIG. 3

FIG. 4

FIG. 5

FIG. 6a

FIG. 6b